(19) Europäisches Patentamt
European Patent Office
Office européen des brevets

(11) Publication number: **0 266 217 B1**

(12) # EUROPEAN PATENT SPECIFICATION

(45) Date of publication of patent specification: **30.12.92**

(51) Int. Cl.[5]: **C12P 7/62**, C12P 13/00, C12P 41/00

(21) Application number: **87309625.9**

(22) Date of filing: **30.10.87**

(54) **Process for producing optically active compounds.**

(30) Priority: **30.10.86 JP 256952/86**
**07.11.86 JP 263837/86**

(43) Date of publication of application:
**04.05.88 Bulletin 88/18**

(45) Publication of the grant of the patent:
**30.12.92 Bulletin 92/53**

(84) Designated Contracting States:
**DE FR GB**

(56) References cited:
**EP-A- 0 064 855**
**EP-A- 0 126 416**

**J. AM. CHEM. SOC., no. 107, 1985, pages 7072-7076, American Chemical Society; G. KIRCHNER et al.: "Resolution of racemic mixtures via lipase catalysis in organic solvents"**

**J. AM. CHEM. SOC., no. 106, 1984, pages 2687-2692, American Chemical Society; B. CAMBOU et al.: "Preparative production of optically active esters and alcohols using esterase-catalyzed stereospecific transesterification in organic media"**

**PROC. NATL. ACAD. SCI. USA, vol. 82, May 1985, pages 3192-3196; A. ZAKS et al.: "Enzyme-catalyzed processes in organic solvents"**

(73) Proprietor: **CHISSO CORPORATION**
**6-32, Nakanoshima 3-chome Higashi-ku**
**Osaka-shi Osaka 530(JP)**

(72) Inventor: **Yoshida, Naoyuki**
**14-15, Deiki 1-chome Kanazawa-ku**
**Yokohama-shi Kanagawa-ken(JP)**
Inventor: **Morita, Hiroshi**
**10-1-401, Seto Kanazawa-ku**
**Yokohama-shi Kanagawa-ken(JP)**

(74) Representative: **Ruffles, Graham Keith et al**
**MARKS & CLERK 57-60 Lincoln's Inn Fields**
**London WC2A 3LS(GB)**

EP 0 266 217 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for producing optically active compounds by a biochemical method in which specific compounds having hydroxyl groups are reacted with esters in the presence of enzymes.

Optically active compounds represented by the general formula (I)

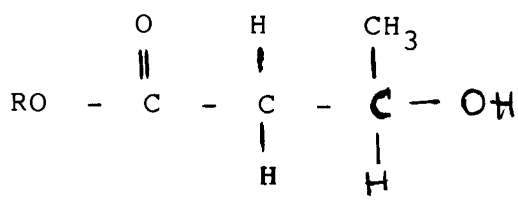

are useful chemical compounds as starting materials for pharmaceuticals, agricultural chemicals and the like, and as intermediates. (In formula I, R is a $C_1$-$C_{20}$ alkyl group).

However, the compounds have optical isomers, and in many cases they do not sufficiently exhibit their useful characteristics unless the R- or S-alcohol is preponderant.

For the above reasons, and in order to obtain an optically active substance, it is necessary to optically resolve a racemate (itself typically obtained by a synthetic chemical technique), to conduct an asymmetric synthesis, or to convert from an optically active starting material by a stereochemical synthetic method. In many cases, the process is troublesome and disadvantageous industrially.

Accordingly, it is desired to develop a technique for obtaining optically active compounds by an industrially advantageous method.

As a known biochemical technique, for example, there is a method described in Japanese Publication of Unexamined Patent Application No. 59-205989 in which a racemic ester is hydrolyzed with a lipase and a desired alcohol is obtained. In this case, the racemic ester is often insoluble in water, so that it is necessary to emulsify or stir vigorously, using a large quantity of water. Klibanov et al. reported a method in which enzyme powder was directly added into a reaction system (J Am Chem Soc, 107, 7072 (1985)). In this case, esters for transesterification are extremely limited and 2,2,2-trichloroethyl butyrate is used as the ester. Furthermore, it is essential to use an organic solvent, such as heptane or ether, which has many problems when it is used industrially.

The inventors of the present invention carried out research for resolving the above problems and obtaining a process for producing optically active compounds represented by the above general formula (I) by an advantageous industrial method. They found that racemic compounds of raw materials can be efficiently resolved to optically active compounds and their antipodes, namely optically active esters, by a biochemical transesterification reaction.

Namely, the present invention provides a process for producing an optically active compound and its ester which comprises using an enzyme having the ability to catalyse preferentially a transesterification reaction between a corresponding ester and an (R,S)-alcohol represented by the above formula (I), reacting the (R,S)-alcohol and the corresponding ester to perform the transesterification reaction under substantially anhydrous conditions, and obtaining an optically active compound which contains richly either the R- or S-alcohol and correspondingly the ester of the S- or R-alcohol.

According to the method of the present invention in comparison with conventional methods, the reaction is conducted under anhydrous conditions. This method does not require the use of a small amount of water or a lower alcohol instead of the water, so that the enzyme is stably kept in organic solvent and easily separated after the reaction and re-used. Furthermore, as the method of the present invention can be kept free from contamination by unwanted microorganisms, there is no necessity for preparing special equipment, antiseptics, sterilization treatment, etc. It is possible to conduct the reaction in an open system. Further, the reaction may be conducted in the same or less quantity of solvent in comparison with common organic synthetic reactions in high substrate concentration.

The following description illustrates this invention more specifically.

In this invention, the (R,S)-alcohols of the raw materials are compounds which are available or can be synthesized.

It is also enough to use esters, preferably triglycerides, which are commercially available without any difficulty. Triacetin, tripropionin, tributyrin, tristearin, trilaurin, trimyristin, triolein, etc, can be exemplified as the triglycerides. As for other ester, methyl propionate, ethyl butyrate, ethyl stearate, trichloroethyl laurate,

butyl laurate, ethylene glycol diacetate, etc, can be used.

As the enzyme which is used in this invention, a lipase, lipoprotein lipase, esterase, etc, is preferable. The enzyme has the ability to catalyse a transesterification reaction preferentially between the R- or S-alcohol and the ester when the enzyme is used with the (R,S)-alcohol, and the enzyme can be used regardless its class. The following table shows commercially available enzymes that can be used in the present reaction.

Table

| Trade name | Origin | Seller or Maker |
|---|---|---|
| Lipase AP | Aspergillus niger | Amano Pharmaceutical Co., Ltd |
| Lipase M | Mucor javanicus | " |
| Lipase P | Pseudomonas fluorescens | " |
| Lipase CES | Pseudomonas sp | " |
| Lipase CE | Humicola lanuginosa | " |
| Lipase F-AP | Rhizopus javanicus | " |
| Lipase II | Porcine Pancreas | Sigma Chemical Co. |
| Lipase VIII | Geotrichum Candidum | " |
| Lipase X | Rhizopus delamar | " |
| Lipase | Chromobacterium Viscosum | Toyo Jozo Co., Ltd. |
| Palatase A | Aspergillus niger | Novo Industi A/S |
| Lipase | Rhizopus niveus | Nagase Biochemicals, Ltd. |

In addition to these enzymes, microorganisms which produce the enzymes having the above ability can be used regardless of their species and genus. As such microorganisms, the genera Arthrobacter, Acromobacter, Alcaligenes Aspergillus, Chromobacterium, Candida, Mucor, Pseudomonas, Rhizopus, etc., can be exemplified.

In practice of the present invention, (R,S)-compounds and esters such as triglycerides can be used without any particular treatments.

The reaction is typically conducted by mixing an (R,S)-compound with an ester, preferably a triglyceride, if necessary adding an organic solvent such as heptane or toluene when the ester is slightly soluble in the compound, and contacting efficiently the mixture with an enzyme. The reaction temperature is suitable 20 to 70 ° C and especially preferably 30 to 45 ° C. The reaction time is widely variable, say 5 to 2000 hours. The reaction time can be shortened by elevating the reaction temperature or lowering the substrate concentration.

The (R,S)-compound which is a substrate and the ester are suitably mixed in the ratio 1:0.5 to 1:5 by mole, and preferably 1:1.1 to 1:2 by mole.

After the transesterification reaction, the enzyme can be removed by conventional filter operation and used again, as it is. The filtrate can be separated into an optically active compound and an ester, respectively, for instance by distillation or column chromatography. The obtained ester is hydrolyzed in an alkali or acid solution to derive the optically active compound which is an antipode of the above compound.

By the above process, the optically active R- and S-compound can be obtained.

The effects of this invention are as follows.

(1) Unnecessary hydrolysis of esters scarcely occurs because the transesterification reaction is substantially conducted under the conditions of no water.

(2) The enzyme can be easily recovered and re-used.

(3) No special equipment and material are used because the reaction can be performed under the conditions of relatively lower temperatures and an open system.

(4) Optically active substances having high purity are obtained by a one-step reaction.

(5) In spite of the biochemical reaction, the substrate concentration can be increased and big reaction vessels are unnecessary, because a buffer solution and the like are not required in the reaction.

Description of Preferred Embodiments

The following examples illustrate this invention more specifically, but these will not always be precise in practical applications.

Reference Example 1

Twenty grammes of enzyme (produced by Amano pharmaceutical Co. Ltd., lipase "Amano p"), 48.9g (0.4mol) of (R,S)-α-phenylethyl alcohol and 133.0g (0.44mol) of tributyrin were charged into three-necked flask and reacted with stirring for nine days at 35 °C. After the reaction was stopped, the enzyme was removed by filtration and the desired compounds were isolated from the filtrate by distillation under reduced pressure. As the result, 28.4g of S-(-)-α-phenylethyl alcohol (yield: 58%, $[\alpha]_D$ = -35.1 ° (neat)) was obtained at the boiling point of 52-62 °C/3mmHg, and R-(+)-1-phenylethyl butyrate was obtained. By hydrolysis of the ester, 18.3g of R-(+)-α-phenylethyl alcohol (yield: 38%, $[\alpha]_D$ = +41.2 ° (neat)) was obtained.

The obtained compounds were identified by structure analysis with NMR.

Example 1

Sixty-six grammes (0.5mol) of racemic ethyl β-hydroxybutyrate, 166.3g (0.6mol) of tributyrin, and 80g of lipase "Amano P" were mixed and reacted with stirring for ten days at 35 °C. After the lipase was removed by fillration, the reaction was stopped.

The filtrate was distilled under vacuum. 14.7g of S-(+)-ethyl β-hydroxybutyrate (yield: 44.5%) ($[\alpha]_D$ = +17.3 ° (c1.0, $CHCl_3$)) and 16.3g of R-(-)-ethyl β-butyryloxybutyrate (yield: 32%) ($[\alpha]_D$ = -0.3 ° (c1.0, $CHCl_3$)) were separated, respectively.

Example 2

Using the same method as described in Reference Example 1, 37.6g (0.235 mol) of racemic tert-butyl β-hydroxybutyrate was resolved. 7.8g of S-(+)-tert-butyl β-hydroxybutyrate (yield: 41%) ($[\alpha]_D$ = +28.2 ° (c1.0, $CHCl_3$)) and 16.2g of R-(-)-tert-butyl β-butyryloxybutyrate (yield: 60%) ($\alpha_D$ = -0.07 ° (neat, 1cm cell)) were separated, respectively.

Further, the obtained R-(-)-tert-butyl β-butyryloxybutyrate was hydrolyzed, and R-(-)-tert-butyl β-hydroxybutyrate ($[\alpha]_D$ = -33.6 ° (c1.0, $CHCl_3$), 95%ee) was obtained. The obtained butyrate was reduced to 1,3-butanediol. The diol was acetylated. The optical purities of the obtained 1,3-diacetyloxybutane was checked with HPLC by using CHIRAL CEL OB.

## Claims

1. A process for producing an optically active compound which comprises using an enzyme which catalyses preferentially a transesterification reaction between an ester and a compound represented by the general formula:

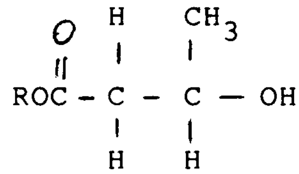

wherein R is an alkyl group having from 1 to 20 carbon atoms, reacting under substantially anhydrous conditions the said (R,S)-compound and the ester to effect the transesterification reaction and obtaining a mixture enriched in either the R- or S- compound.

**2.** A process as claimed in claim 1, wherein the starting ester is triglyceride.

**Patentansprüche**

**1.** Ein Verfahren zum Herstellen einer optisch aktiven Verbindung, welches umfaßt: Anwendung eines Enzymes, das vorzugsweise eine Umesterungsreaktion zwischen einem Ester und einer durch die allgemeine Formel

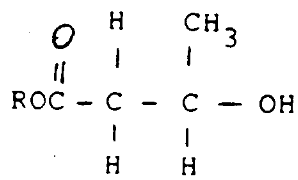

dargestellten Verbindung katalysiert, worin R eine Alkylgruppe mit 1 bis 20 Kohlenstoffatomen ist, Umsetzung besagter (R, S)-Verbindung und des Esters unter im wesentlichen wasserfreien Bedingungen, um die Umesterungsreaktion zu bewirken, und Gewinnung einer entweder mit der R- oder der S-Verbindung angereicherten Mischung.

**2.** Verfahren nach Anspruch 1, bei dem der Ausgangsester Triglycerid ist.

**Revendications**

**1.** Un procédé pour la préparation d'un composé optiquement actif qui comprend l'utilisation d'une enzyme qui catalyse de préférence une réaction de transestérification entre un ester et un composé représenté par la formule générale:

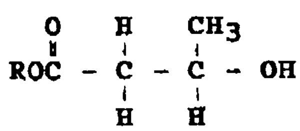

dans laquelle R est un groupement alkyle ayant de 1 à 20 atomes de carbone, la réaction dans des conditions substantiellement anhydres dudit composé (R,S) et de l'ester pour effectuer la réaction de transestérification et l'obtention d'un mélange enrichi soit en composé R- soit en composé S-.

**2.** Un procédé tel que revendiqué dans la revendication 1, dans lequel l'ester de départ est un triglycéride.